# EUROPEAN PATENT SPECIFICATION

(11) **EP 2 908 756 B1**
(45) Date of publication and mention of the grant of the patent: **13.11.2019**
(21) Application number: 13729421.1
(22) Date of filing: 12.03.2013
(51) Int. Cl.: A61B 18/04, A61N 1/32, A61B 18/14

(54) **ELECTROMEDICAL DEVICE**
ELEKTROMEDIZINISCHE VORRICHTUNG
DISPOSITIF ÉLECTROMÉDICAL

(30) Priority: 17.10.2012 IT RM20120503
(43) Date of publication of application: 26.08.2015
(73) Proprietor: GMV S.r.l., 00173 Roma (IT)
(72) Inventor: MILLEVOLTE, Giancarlo, I-00040 Rome (IT); FIPPI, Giorgio, I-00122 Rome (IT)
(74) Representative: Romano, Giuseppe
(86) International application number: PCT/IB2013/051948
(87) International publication number: WO 2014/060854

(56) References cited:
- WO-A2-2011/055368
- WO-A2-2012/106735
- US-A1- 2003 125 727
- US-A1- 2011 301 412

## Description

### Technical Field of the Invention

The present invention refers to an electromedical device, in particular for non-ablative plastic surgery, for treating unaesthetisms and skin pathologies.

### Background

To date, it is felt the need to intervene on pathologies in which laser or radio-wave scalpel use proves difficult, as those of skin tissues; the latter conduct badly, both thermally and electrically, and have always been an obstacle to perfect performing of the most common dermatological surgery interventions, as a diathermal current follows the shortest, and usually undesired electrical path.

Moreover, the depolarization of free nerve endings causes an unpleasant feeling of electrical discharge, making local anesthesia necessary. WO2011/055368 discloses a medical device for tissue welding comprising a supply unit connected to a hand-held plasma head via a flexible hose for supplying the plasma head with a gas and energy for exciting the gas and creating the plasma.

US2003/0125727 discloses a medical instrument coupled to first and second energy means and a computer controller for the controlled volumetric removal of thin tissue layers using a source for introducing a gas.

### Summary of the Invention

Therefore, object of the present invention is to overcome the above-disclosed issues, and this is attained by an electromedical device as set forth by claim 1.

The present invention, by overcoming the problems of the known art, entails numerous evident advantages.

In particular, the device according to the present invention, due to how it is made, allows to avoid possible electrical discharges due to the passage of current toward the patient. For these reasons, it proves to be the ideal instrument for treating sensitive zones, like the periocular one in a blepharoplasty intervention, with no risk of damage to the optic nerve.

Moreover, the treated portion cools automatically by evaporation of lesional fluids during the intervention, not overheating the surrounding tissues.

Moreover, it reduces patient's perception of discomfort and pain, as advantageously the operator, energies output by the instrument being equal, can vary the applying time on the zone of interest; therefore, it is possible to effectively treat also delicate and sensitive zones, such as mucosae and genitals.

The electromedical device is of wireless type - i.e. without electrical transmission cables - practical, handy, reliable and provided with a portable charging station.

A further advantage with respect to the known art is that the device according to the present invention is characterized by the absence of the reference plate and moreover, by utilizing a high-voltage, low-amperage sinusoidal signal, with a constant output control system, it allows to prevent possible electric discharges, since - as already mentioned - there is no passage of current toward the patient.

These and other advantages, along with the features and the modes of employ of the present invention, will be made apparent in the following detailed description of preferred embodiments thereof, given by way of example and not for limitative purposes.

### Brief Description of the Figures

Reference will be made to the figures of the annexed drawings, wherein:
- Figure 1 shows a perspective view of a preferred embodiment of the device according to the present invention;
- Figures 2 and 3 show a perspective view of a preferred embodiment of a system, respectively in a closed configuration and in an open configuration, comprising the device of Figure 1;

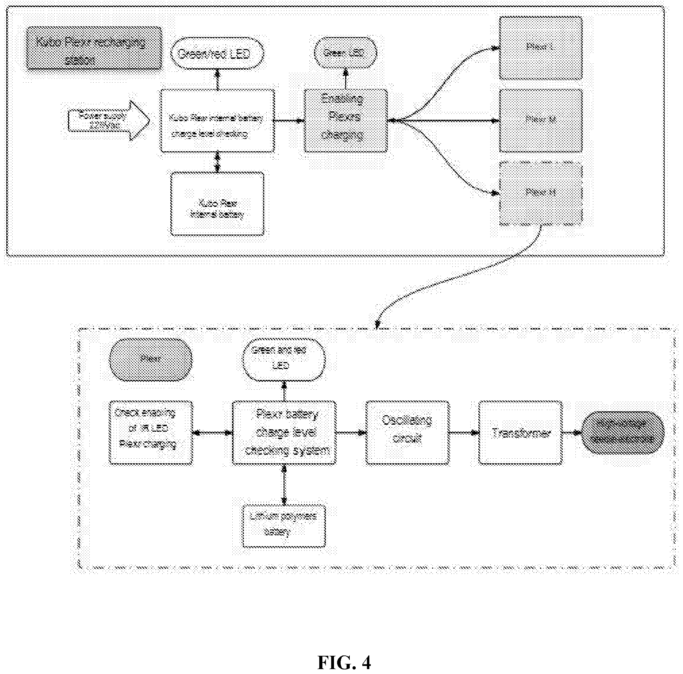

- Figure 4 shows an operation block diagram of the system of Figure 2;
- Figure 5 shows a representative image of a mode of use of the device of Figure 1.

### Detailed description of preferred embodiments

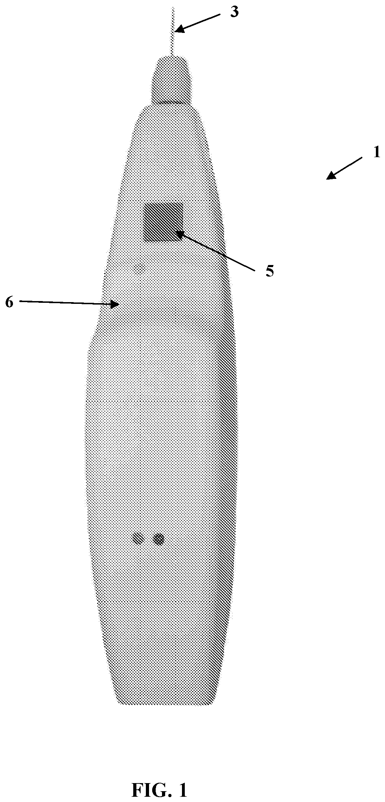

Referring initially to Figure 1, an electromedical device for non-ablative plastic surgery according to a preferred embodiment of the invention is generally denoted by 1.

In the present embodiment, the electromedical device is intended for treating unaesthetisms and/or pathologies of a skin portion.

Therefore, in Figure 1 a holding means 6 is depicted, shaped to be grasped by an operator; in particular, the handle is ergonomically shaped. The device 1 further comprises an electrode 3, removably mounted or mountable to the holding means 6 and having a free end.

In particular, the electrode is mounted, e.g., via the use of a mandrel 4, used to lock in the correct position the electrode shaped as a needle.

Moreover, though not visible in the figures, the device 1 comprises electric generator means, apt to generate an electric signal to be applied to the above-mentioned electrode 3. The electric signal applied to the electrode 3 is apt to ionize a gas at the free end of the electrode, so that, in an operative condition wherein the same electrode is in proximity to skin portion to be treated, the abovementioned ionization is so to heat the skin portion to be treated.

In particular, as visible in the figures, the electrode 3 is shaped as a needle.

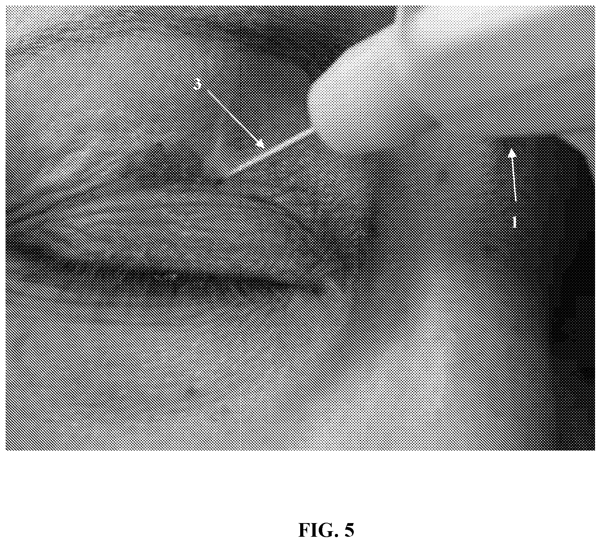

As shown in Figure 5, the device 1, through a needle, is capable of transferring concentrated energy with a high-voltage, low-amperage electron flow on the tissues to be treated. The electrodes are comprised of the needle and the patient's tissue. The device can be utilized both on badly conducting tissues and on normally conducting tissues (e.g.: skin spots, naevi, condylomas, fibromas, wrinkles), simply by varying the distance between tip and skin, without causing dips and dyschromias. Advantageously, moreover, the treated part cools down automatically by evaporation of lesional fluids during the intervention, without overheating the surrounding tissues.

This instrument, dedicated to Specialists in the field, joins and collaborates with laser, radio-wave scalpel, felc (conveyed electron flow), pulsed light, radiofrequency. Advantageously, the electromedical device utilizes the fourth state of matter: PLASMA. Treated tissues sublimate, thereby preventing transfer of undesired quantities of heat to surrounding zones and subcutaneous region.

In the present example, the electric generator means comprises a battery, in particular a lithium polymers battery. Moreover, means for indicating the battery charge is provided, in particular luminous indicators. For instance, the use of green/red LED elements apt to change color according to the battery state of charge is provided. In particular, the battery charging process is managed by infrared LEDs. Upon restoring the correct battery charge level of the device 1, the IR led disconnects from the charging device 10, as better described hereinafter.

In the preferred embodiments, the electric generator means further comprises an oscillating circuit for generating a sinusoidal electric signal.

Preferably, the electric signal, and in particular the sinusoidal signal, is a signal having a power value in the range of 0.7 W to 2 W.

In particular, the signal is of sinusoidal type, with an operating voltage of 750Vac, a Frequency of 50kHz±25% and a maximum power emitted of 2W.

According to the specific intervention and the specific skin pathology to be treated, it is in fact possible to change the treating power.

The operation principle of the device 1 may be summed up into three basic steps:
- potential difference between the device 1 and the skin portion to be treated.
- formation of a small electric arc, similar to a tiny flash of lighting, due to ionization of air-contained gases (Plasma generation).
- surface tissue sublimation, without undesired transfers of heat to other tissues.

In particular, the operation principle of the device 1 is based on the ionization of a gas, in particular of air about the electrode tip.
In particular, the electric generator means comprises a transformer apt to raise the voltage of the electric signal at said electrode 3. E.g., a voltage value so as to ionize air surrounding the free end itself is reached at the free end of the electrode shaped as a needle.

Advantageously, adjusting means for adjusting the supply of the electric signal is provided. In particular, a switch 5 for switching on and off the supply is positioned at the handle, as shown in Figure 1.

In a further preferred embodiment, the adjusting means is programmable, for instance from the outside via a suitably configured user interface, so as to establish, prior to the actual operativeness of the device, the strength and the duration of the supply cycles of the electric signal.

To facilitate a constant lighting of the skin portion to be treated, the holding means 6 comprises a light source, in particular placed at a mounting region of the electrode. In particular, it is provided the use of a lighting element colored with a different color (e.g., red , black, or green) according to the energy used.

Moreover, a still further embodiment of the present invention provides an electromedical system 11 for non-ablative plastic surgery, comprising one or more devices 1 as described above and a charging device 10 apt to recharge the devices themselves.

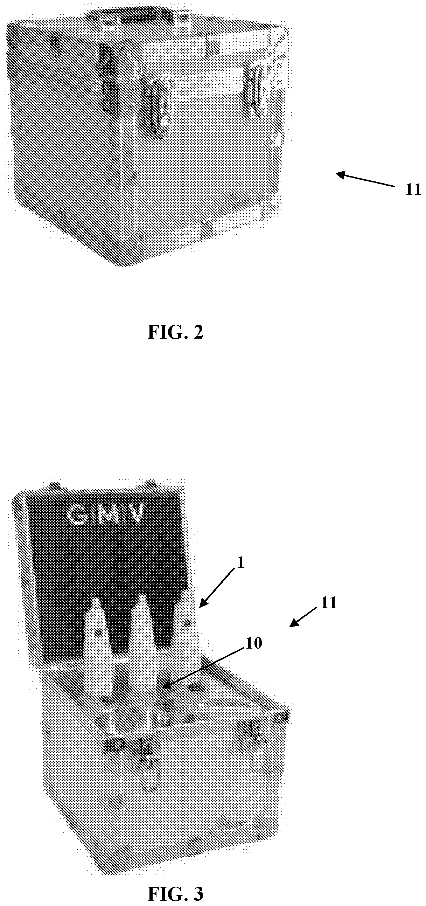

As shown in Figures 2 and 3, the system 11 preferably comprises three devices 1 apt to generate a signal, e.g. respectively at powers of 0.7W, 1W and 2W, so as to effectively treat unaesthetisms different from each other.

The charging device 10 comprises a further battery apt to recharge the batteries of said one or more devices 1. In particular, the further battery is rechargeable by direct connection to the electric network.

The presence of this two-battery recharging system (battery of the device 1 and further battery of the charging device 10) allows to have a certain autonomy from the electric network. In case of blackout the system is oversized with respect to the surgeon's needs, with an autonomy of about 48 hours.

The electromedical system 11 further comprises processing means apt to program the above-mentioned programmable adjusting means for adjusting the supply of said electric signal. E.g., the use of a touch screen-type user interface is provided to allow said programming.

In particular, the device at a low power, e.g. 0.7W, is utilized for unaesthetisms such as blepharoplasty, ring-block anesthesia, skin spots, small soft warts, active acne, wrinkles, periumbilical stretch marks, xanthelasmas, small neoformations or tattoos. The use of the device 1 at a medium power, e.g. 1W, is for active acne, wrinkles, medium neoformations, small keloids, small ruby angiomas, face lifting, significant xanthelasmas with thickness.

The device at a high power, e.g. 2W, is utilized for large moles, keloids, medium/large ruby angiomas, seborrheic warts, large fibromas.

In an operative stage, it is possible to work, e.g., in a single-spot mode, for instance with spots set triangle-like, with an overcasting (zigzag) technique, preferably on areas in relief and not on the bottom of wrinkles, furrows and scars, in order to attain a lifting effect.

Alternatively, it is possible to work in a spray mode, i.e. uniformly over the entire skin surface to be treated.

Hereinafter, an exemplary case is reported, carrying some indications to be taken into account in use of a preferred embodiment of the device 1 according to the present invention:
1. Firmly grasp the device.
2. Lean on a bone plane.
3. Press for required time only.
4. Keep the electrode shaped as a needle always at the right distance.
5. Do not exceed 2 seconds per each spot.
6. Disposable electrode.
7. Benzalkonium-based disinfectant.
8. Avoid plasters, creams, medicaments and cosmetics.
9. Protect from UVA- and UVB-rays (nail reconstruction and dentist).

Hereinafter, some exemplary cases of application of a preferred embodiment of the device 1 are reported:

### Blepharoplasty

**General introduction:** Use green Citrosil® as disinfectant - EMLA cream anaesthetic 30 minutes prior to the intervention; remove carefully before starting the intervention.

### Procedure:

1. Gently pinch the part.
2. Intervene by spots, strictly set triangle-like, so as to create force lines, acting throughout 360 degrees, sublimating surface corneocytes.
3. As soon as treatment is over, apply ice for at least 30 minutes.

### For the patient

Wash gently with water and Marseille soap, dry by swabbing without rubbing, treat with benzalkonium chloride-based eyewash (the one best known in Italy is "ALFA C"), along the entire scab occurring period, until all small Carbon scales have come off, then protect from the Sun with a total-protection fluid foundation cream.

### Repeat intervention after 28 days.

### Skin spots

Spots are to be classified into two categories:
- Epidermal spots (those which disappear when swabbing with cotton).
- Dermal spots (do not disappear when swabbing with cotton).

### Procedure:

1. Cover the surface, as if to make a drawing, trying to tone down the spot, as if staining it with Carbon (spray-mode Plexr).
2. As soon as it is over, take a Citrosil®-soaked ball of cotton wool, put the cotton on the spot and wait some seconds, swabbing with to-and-fro motions.

**Repeat intervention after 28 days,** until spot disappears completely.

Never try to remove it with a single session.

**For the patient:** protect it with a total-protection fluid foundation cream.

### Achromatic spots (white spots)

### Procedure:

1. Treat them in concentric spirals, starting from a circumference external to the spot until covering all of it.
2. Do not apply Citrosil®; the patient will disinfect them the following day. Never use protection; actually, Sun exposure is to be preferred.

### Xanthelasms

### Procedure:

- Treat the entire surface, each time removing the carbonaceous deposits and repeating the operation several times, up to disappearance.
- Cool down the part; at home, disinfect with eyewash.
- Check after 28 days.

### Active acne

### Procedure:

1. Sublimate transition ring between yellow part and red part.
2. Of the millet seed white zone, only the part at the center of the "millet seed" has to be removed.

### Post-acne and Varicella (chickenpox) scars

### Procedure:

1. Treatment in spots on bumps between a scar and the other one, always triangle-like, getting to the scar edge.
2. This treatment can be carried out on edges of glabellar wrinkles, and anywhere skin has to be shortened.
3. Treatments are to be carried out every 28 days.
4. Disinfect and apply foundation cream.

### Warts - Fibromas - Moles and Neoformations in Plus

### Procedure:

1. Ring-block anesthesia with the device low on the entire contour, in a continuous fashion.
2. Then, apical part is tackled until disappearance.
3. Check after 28 days.
4. Disinfect and protect.

### Expression wrinkles (bar code)

### Procedure:

1. Act on edges, never on bottom.
2. If wrinkles are very deep, spread out tissue between wrinkles by the triangle technique in order to shorten the skin.
3. Disinfect and protect with total-protection fluid foundation cream.
4. Check after 28 days.

In particular, hereinafter there are reported the specifications, carrying parameters of a preferred embodiment of the system 11 according to the invention:

| **System parameters** | **Values** |
|---|---|
| Device 1 SIZE | H 16.5cm, L 3.5cm, D 3cm |
| Charging device 10 SIZE | H 26.5cm, L 26.5cm, D 26.5cm |
| Device 1 POWER SUPPLY | Built-in Lithium polymers battery, 12V, 450mAh |
| | LIPOL battery A 11V |
| Induction BATTERY CHARGER, internal to charging device 10 | Power supply: 220V- 50/60Hz |
| | Operating voltage: max. 80V |
| | Output frequency: 830kHz |
| | Max. output power: 20VA |
| MAX OPERATING VOLTAGE | 750 Vac |
| OUTPUT FREQUENCY | 50kHz±25% |
| MAX OUTPUT POWER | ≤ 2W ±10% |
| Plexr® Power: High (Red) | ≤ 2W ±10% |
| Plexr® Power: Medium (Green) | 1W ± 10% |
| Plexr® Power: Low (White) | 0.7W ±10% |
| ELECTRODE for applying interchangeable needle | Disposable, sterile, of stainless steel (manufactured by Bionen sas, Florence) |
| CERTIFICATION | EC, according to directive 93/42/EEC |
| CLASSIFICATION | IIb |

As to the construction features of the device 1 and of the system 11, preferably the charging device 10 is shaped like a box complete with a front cover, e.g. made of anticorodal, containing electronics required for the various processes for checking the batteries charge level and being equipped with connectorized-type inlets and outlets from a panel for power supplying from a network.

In the preferred configuration shown in Figures 2 and 3, the charging device 10 has three distinct housings for the process of recharging the three devices 1.

Moreover, some exemplary parameters concerning safety regulations are reported hereinafter:
- The apparatus should be subjected to electrical safety tests and electromagnetic compatibility tests envisaged by the following harmonized technical Standards:
- EN 60601-1:2006 Medical electrical equipment - Part 1: General requirements for basic safety and essential performance.
- EN 60601-1-2:2007 Medical electrical equipment - Part 1: General requirements for basic safety and essential performance - Collateral standard: Electromagnetic compatibility - Requirements and tests
- The components related to network power supply should be of medical standard and shall be subject to traceability.

The present invention has hereto been described with reference to preferred embodiments thereof. It is to be understood that each of the technical solutions implemented in the preferred embodiments described hereto by way of example could advantageously be combined thereamong in a different manner to originate other embodiments, all afferent to the same inventive kernel and all anyhow falling within the protective scope of the appended claims.

## Claims

1. Electromedical device (1) without electrical transmission cables for non-ablative plastic surgery, in particular apt for treating unaesthetisms and/or pathologies of a skin portion of a patient, comprising:
- holding means (6) shaped to be grasped by an operator;
- a needle-shaped electrode (3), removably mounted or mountable to said holding means (6) and having a free end;
- electric generator means, comprising an oscillating circuit for generating a sinusoidal electric signal having a power value in the range of 0.7 W to 2 W, with an operating voltage of 750 Vac and a frequency of 37.5 kHz to 62.5 kHz, to be applied to said electrode;
said electric signal applied to the electrode being apt to ionize air at the free end of said electrode,
the device not having a reference plate to be applied to the skin of the patient for closing the electric circuit of the electrode.

2. Electromedical device (1) according to any one of the preceding claims, wherein said electric generator means comprises a battery, in particular a lithium polymers battery.

3. Electromedical device (1) according to any one of the preceding claims, wherein said electric generator means comprises a transformer apt to raise the voltage of the electric signal at said electrode (3).

4. Electromedical device (1) according to any one of the preceding claims, further comprising adjusting means for adjusting the supply of said electric signal.

5. Electromedical device (1) according to the preceding claim, wherein said adjusting means is programmable.

6. Electromedical device (1) according to any one of the preceding claims, wherein said holding means (6) comprises a light source, in particular placed at a mounting region of said electrode.

7. Electromedical device (1) according to any one of the preceding claims, further comprising means for indicating the battery charge.

8. Electromedical device (1) according to the preceding claim, wherein said indicating means are LEDs.

9. Electromedical system (11) for non-ablative plastic surgery, comprising one or more devices (1) according to any one of the claims 1 to 8 and further comprising a charging device (10) apt to recharge said one or more devices (1).

10. Electromedical system (11) according to the preceding claim, wherein said charging device (10) comprises a further battery apt to recharge the batteries of said one or more devices (1).

11. Electromedical system (11) according to any one of the claims 9 to 10, when dependent from claim 5, further comprising processing means apt to program said programmable adjusting means for adjusting the supply of said electric signal.

## Patentansprüche

1. Elektromedizinische Vorrichtung (1) ohne elektrische Übertragungskabel für die nicht ablative plastische Chirurgie, im Besonderen geeignet zum Behandeln von unästhetischen und/oder pathologischen Erscheinungen einer Hautpartie eines Patienten, die umfasst:
- Haltemittel (6), das so geformt ist, dass es durch einen Anwender erfasst werden kann;
- eine nadelförmige Elektrode (3), die an das Haltemittel (6) abnehmbar angebracht oder anbringbar ist und über ein freies Ende verfügt;
- Stromgeneratormittel, das umfasst: einen Schwingkreis zum Erzeugen eines sinusförmigen elektrischen Signals, das über einen Leistungswert in dem Bereich von 0,7 W bis 2 W verfügt, mit einer Betriebsspannung von 750 Vac und einer Frequenz von 37,5 kHz bis 62,5 kHz, das auf die Elektrode anzuwenden ist;
wobei das auf die Elektrode anzuwendende Signal geeignet ist, Luft am freien Ende der Elektrode zu ionisieren,
wobei die Vorrichtung über keine Referenzplatte verfügt, die auf die Haut des Patienten zum Schließen des Stromkreises der Elektrode anzuwenden ist.

2. Elektromedizinische Vorrichtung (1) gemäß einem der vorangehenden Ansprüche, wobei das Stromgeneratormittel umfasst: eine Batterie, im Besonderen eine Lithium-Polymer-Batterie.

3. Elektromedizinische Vorrichtung (1) gemäß einem der vorangehenden Ansprüche, wobei das Stromgeneratormittel umfasst: einen Transformator, der geeignet ist, die Spannung des elektrischen Signals bei der Elektrode (3) zu erhöhen.

4. Elektromedizinische Vorrichtung (1) gemäß einem der vorangehenden Ansprüche, die weiterhin umfasst: Einstellmittel zum Einstellen der Bereitstellung des elektrischen Signals.

5. Elektromedizinische Vorrichtung (1) gemäß dem vorangehenden Anspruch, wobei das Einstellmittel programmierbar ist.

6. Elektromedizinische Vorrichtung (1) gemäß einem der vorangehenden Ansprüche, wobei das Haltemittel (6) umfasst: eine Lichtquelle, die im Besonderen bei einem Befestigungsbereich der Elektrode angeordnet ist.

7. Elektromedizinische Vorrichtung (1) gemäß einem der vorangehenden Ansprüche, die weiterhin umfasst: Mittel zum Anzeigen der Batterieladung.

8. Elektromedizinische Vorrichtung (1) gemäß dem vorangehenden Anspruch, wobei die Anzeigemittel LEDs sind.

9. Elektromedizinisches System (11) für die nicht ablative plastische Chirurgie, das eine oder mehrere Vorrichtungen (1) gemäß einem der Ansprüche 1 bis 8 umfasst, und weiterhin umfasst: eine Ladevorrichtung (10), die geeignet ist, die eine oder die mehreren Vorrichtungen (1) wieder aufzuladen.

10. Elektromedizinisches System (11) gemäß dem vorangehenden Anspruch, wobei die Ladevorrichtung (10) umfasst: eine weitere Batterie, die geeignet ist, die Batterien der einen oder mehreren Vorrichtungen (1) wieder aufzuladen.

11. Elektromedizinisches System (11) gemäß einem der Ansprüche 9 bis 10, das, wenn in Abhängigkeit von Anspruch 5, weiterhin umfasst: Verarbeitungsmittel, das geeignet ist, das programmierbare Einstellungsmittel zum Einstellen der Bereitstellung des elektrischen Signals zu programmieren.

## Revendications

1. Dispositif électromédical (1) sans câbles de transmission électrique pour chirurgie plastique non ablative, en particulier apte à traiter des aspects inesthétiques et/ou des pathologies d'une partie de la peau d'un patient, comprenant :
- un moyen de préhension (6) mis en forme pour être saisi par un opérateur ;
- une électrode en forme d'aiguille (3), montée ou apte à être montée de manière amovible sur ledit moyen de préhension (6) et ayant une extrémité libre ;
- un moyen de générateur électrique, comprenant un circuit oscillant pour générer un signal électrique sinusoïdal ayant une valeur de puissance dans la plage de 0,7 W à 2 W, avec une tension de fonctionnement de 750 Vca et une fréquence de 37,5 kHz à 62,5 kHz, à appliquer à ladite électrode ;
ledit signal électrique appliqué à l'électrode étant apte à ioniser l'air au niveau de l'extrémité libre de ladite électrode,
le dispositif n'ayant pas de plaque de référence à appliquer sur la peau du patient pour fermer le circuit électrique de l'électrode.

2. Dispositif électromédical (1) selon l'une quelconque des revendications précédentes, dans lequel ledit moyen de générateur électrique comprend une batterie, en particulier une batterie lithium-polymères.

3. Dispositif électromédical (1) selon l'une quelconque des revendications précédentes, dans lequel ledit moyen de générateur électrique comprend un transformateur apte à élever la tension du signal électrique au niveau de ladite électrode (3).

4. Dispositif électromédical (1) selon l'une quelconque des revendications précédentes, comprenant en outre un moyen de réglage pour régler l'alimentation par ledit signal électrique.

5. Dispositif électromédical (1) selon la revendication précédente, dans lequel ledit moyen de réglage est programmable.

6. Dispositif électromédical (1) selon l'une quelconque des revendications précédentes, dans lequel ledit moyen de préhension (6) comprend une source de lumière, en particulier placée au niveau d'une région de montage de ladite électrode.

7. Dispositif électromédical (1) selon l'une quelconque des revendications précédentes, comprenant en outre un moyen pour indiquer la charge de la batterie.

8. Dispositif électromédical (1) selon la revendication précédente, dans lequel ledit moyen d'indication est constitué de DEL.

9. Système électromédical (11) pour chirurgie plastique non ablative, comprenant un ou plusieurs dispositifs (1) selon l'une quelconque des revendications 1 à 8 et comprenant en outre un dispositif de charge (10) apte à recharger lesdits un ou plusieurs dispositifs (1).

10. Système électromédical (11) selon la revendication précédente, dans lequel ledit dispositif de charge (10) comprend une batterie supplémentaire apte à recharger les batteries desdits un ou plusieurs dispositifs (1).

11. Système électromédical (11) selon l'une quelconque des revendications 9 à 10, lorsque dépendante de la revendication 5, comprenant en outre un moyen de traitement apte à programmer ledit moyen de réglage programmable pour régler l'alimentation par ledit signal électrique.
